# EUROPÄISCHE PATENTSCHRIFT

(11) **EP 3 166 705 B1**
(45) Veröffentlichungstag und Bekanntmachung des Hinweises auf die Patenterteilung: **15.08.2018**
(21) Anmeldenummer: 15734382.3
(22) Anmeldetag: 07.07.2015
(51) Int. Cl.: B01D 3/32, B01D 3/40, B01F 5/04, C07C 7/08, C07C 7/167

(54) **KOLONNE MIT TRENNWIRKSAMEN EINBAUTEN ZUR AUFTRENNUNG EINES GEMISCHES VON KOHLENWASSERSTOFFEN UND/ODER KOHLENWASSERSTOFFDERIVATEN DURCH EXTRAKTIVDESTILLATION MIT EINEM SELEKTIVEN LÖSUNGSMITTEL**
COLUMN WITH SEPARATIVE INSTALLATIONS FOR SEPARATING A MIXTURE OF HYDROCARBONS AND/OR HYDROCARBON DERIVATIVES BY MEANS OF AN EXTRACTIVE DISTILLATION USING A SELECTIVE SOLVENT
COLONNE COMPRENANT DES ÉLÉMENTS SÉPARATEURS INTÉGRÉS SERVANT À SÉPARER UN MÉLANGE D'HYDROCARBURES ET/OU DE DÉRIVÉS D'HYDROCARBURES PAR DISTILLATION EXTRACTIVE AU MOYEN D'UN SOLVANT SÉLECTIF

(30) Priorität: 08.07.2014 EP 14176082
(43) Veröffentlichungstag der Anmeldung: 17.05.2017
(73) Patentinhaber: BASF SE, 67056 Ludwigshafen am Rhein (DE)
(72) Erfinder: HEIDA, Bernd, 67158 Ellerstadt (DE); HOFINGER, Julia, 67071 Ludwigshafen (DE); RENZE, Peter, 68163 Mannheim (DE)
(74) Vertreter: BASF IP Association
(86) Internationale Anmeldenummer: PCT/EP2015/065423
(87) Internationale Veröffentlichungsnummer: WO 2016/005359

(56) Entgegenhaltungen:
- DE-A1-102010 011 014
- FR-A1- 2 606 291
- US-A- 2 610 141
- US-A- 4 802 630
- US-A1- 2005 059 838

## Beschreibung

Die Erfindung betrifft eine Kolonne mit trennwirksamen Einbauten zur Auftrennung eines Gemisches von Kohlenwasserstoffen und/oder Kohlenwasserstoffderivaten durch Extraktivdestillation mit einem selektiven Lösungsmittel sowie ein Verfahren unter Verwendung der Kolonne. Zum Beispiel betrifft DE102010011014A1 eine Extraktivdestillation. Extraktivdestillationskolonnen dienen zur Auftrennung von Gemischen von Kohlenwasserstoffen und/oder Kohlenwasserstoffderivaten unter Einsatz eines selektiven Lösungsmittels, das die Flüchtigkeitsunterschiede zwischen den Komponenten des aufzutrennenden Gemisches erhöht. Das aufzutrennende Gemisch und das selektive Lösungsmittel werden im Gegenstrom über die trennwirksamen Einbauten der Kolonne geleitet, wobei sich das selektive Lösungsmittel mit den Komponenten aus dem aufzutrennenden Gemisch belädt, zu dem es eine höhere Affinität hat, und als beladenes selektives Lösungsmittel aus dem unteren Kolonnenbereich abgezogen wird, wogegen die Komponenten aus dem aufzutrennenden Gemisch, zu denen das selektive Lösungsmittel eine niedrigere Affinität hat, in der Dampfphase verbleiben und als Kopfstrom abgezogen werden, der vollständig oder teilweise unter Erhalt eines Kondensats kondensiert wird, das teilweise als Produktstrom abgezogen und im Übrigen als Rücklauf erneut auf die Kolonne aufgegeben wird.

Für einen effizienten Betrieb der Kolonne ist es erforderlich, dass der Rücklauf gleichmäßig mit dem selektiven Lösungsmittel vermischt wird und eingelöst, so dass eine einphasig flüssige Lösung mit homogener Zusammensetzung erhalten wird.

Bei bekannten Kolonnen, ohne die erfindungsgemäße Vorvermischung von selektivem Lösungsmittel und Rücklauf, das heißt mit getrennter Zuführung derselben, verliert die Kolonne beträchtlich an Effizienz, weil die Einlösung und Homogenisierung der beiden Flüssigkeiten zumindest teilweise im oberen Bereich der trennwirksamen Einbauten erfolgt und diese somit nicht für die eigentliche Trennaufgabe zur Verfügung stehen. So liegt der Effizienzverlust für eine Kolonne mit einem Durchmesser von 4,70 m in der Extraktivdestillation zur Abtrennung von 1,3-Butadien aus einem C₄-Schnitt mit N-Methylpyrrolidon als selektivem Lösungsmittel im Bereich von bis zu 60 %.

Zur Lösung des obigen Problems wurde beispielsweise vorgeschlagen, zwei Flüssigkeitsverteiler übereinander anzuordnen, und auf jeden derselben jeweils einen der zu vermischenden Flüssigkeitsströme aufzugeben. Dadurch kann zwar eine teilweise Verbesserung der Effizienz der Kolonne erreicht werden, jedoch unter Inkaufnahme von zusätzlicher Bauhöhe.

Es war demgegenüber Aufgabe der Erfindung, eine Extraktivdestillationskolonne zur Verfügung zu stellen, die eine ausgezeichnete Vermischung von Rücklauf und selektivem Lösungsmittel gewährleiste ohne dass Effizienzverluste oder eine zusätzliche Bauhöhe der Kolonne in Kauf genommen werden müssten.

Die Aufgabe wird gelöst durch eine Kolonne mit trennwirksamen Einbauten zur Auftrennung eines Gemisches von Kohlenwasserstoffen und/oder Kohlenwasserstoffderivaten durch Extraktivdestillation mit einem selektiven Lösungsmittel, mit Zuführung des selektiven Lösungsmittels im oberen Kolonnenbereich und Zuführung des aufzutrennenden Gemisches von Kohlenwasserstoffen und/oder Kohlenwasserstoffderivaten unterhalb der Zuführung des selektiven Lösungsmittels, wobei sich in der Kolonne das selektive Lösungsmittel mit den Komponenten aus dem aufzutrennenden Gemisch belädt, zu denen es eine höhere Affinität hat, und als beladenes selektives Lösungsmittel aus dem unteren Kolonnenbereich abgezogen wird, wogegen die Komponenten aus dem aufzutrennenden Gemisch, zu denen das selektive Lösungsmittel eine niedrigere Affinität hat, in der Dampfphase verbleiben und als Kopfstrom abgezogen werden, der vollständig oder teilweise unter Erhalt eines Kondensats kondensiert wird, das teilweise als Produktstrom abgezogen und im Übrigen als Rücklauf erneut auf die Kolonne aufgegeben wird, die dadurch gekennzeichnet ist, dass
- im Kolonnenbereich oberhalb der trennwirksamen Einbauten, ein erstes, im Wesentlichen horizontales Einleitrohr für die Zuführung des selektiven Lösungsmittels vorgesehen ist,
- wobei das erste, im Wesentlichen horizontale Einleitrohr eine Querschnittsverengung bis zu einer engsten Stelle aufweist, und sich nach der Querschnittsverengung erneut aufweitet, und wobei
- ein zweites Einleitrohr, das im Bereich der engsten Stelle der Querschnittsverengung in das erste, im Wesentlichen horizontale Einleitrohr einmündet, für die Zuführung des Rücklaufs vorgesehen ist.

Ein Fachmann auf dem Gebiet der Extraktivdestillationen hätte eine Einmischvorrichtung für den Rücklauf in das selektive Lösungsmittel in der obigen Form, die den Venturi-Effekt nutzt, nicht eingesetzt, insbesondere wegen der Befürchtung, dass der Treibstrahl, vorliegend der Strom des selektiven Lösungsmittels, Gas einsaugen könnte, wobei sich Gaspolster bilden können, die sich negativ auf die Gleichverteilung der Flüssigkeit über den Kolonnenquerschnitt auswirken.

Es hat sich jedoch überraschenderweise gezeigt, dass sich im Bereich der engsten Stelle der Querschnittsverengung des ersten, im Wesentlichen horizontalen Einleitohres ein dynamisches Gleichgewicht einstellt, das im Wesentlichen wie folgt beschrieben werden kann:
Die Saugwirkung des Flüssigkeitsstrahles des selektiven Lösungsmittels, das durch das erste, im Wesentlichen horizontale Einleitrohr einströmt erlischt, sobald über das zweite Einleitrohr, das im Bereich der engsten Stelle der Querschnittsverengung in das erste, im Wesentlichen horizontale Einleitrohr einmündet, eine bestimmte Menge Gas eingesaugt wird; es staut sich eine Flüssigkeitssäule im zweiten Einleitrohr auf, die aber sehr bald wieder zerstört wird, da sich die Saugwirkung erneut einstellt, sobald auch nur eine geringe Flüssigkeitssäule im zweiten Einleitrohr aufgebaut ist. Das bereits eingesaugte Gas wird aufgrund der hohen Turbulenzen abwärts der engsten Stelle der Querschnittsverengung vollständig in die Flüssigkeit eingelöst.

Erfindungsgemäß kann jede bekannte Extraktivdestillationskolonne eingesetzt werden. Bevorzugt sind Kolonnen im World-Scale Maßstab, weil bei großen Kolonnendurchmessern, insbesondere von größer als 0,5 m oder auch von größer als 1,5 m, die Einlösung und Homogenisierung des Rücklaufs mit dem selektiven Lösungsmitteln mit zunehmendem Kolonnendurchmesser schwieriger wird. Die trennwirksamen Einbauten können insbesondere Böden oder Packungen sein. Im Falle von Packungen sind Flüssigkeitsverteiler oberhalb derselben stets erforderlich.

Vorteilhaft ist oberhalb der trennwirksamen Einbauten in der Kolonne ein Flüssigkeitsverteiler angeordnet.

Im Falle von Böden als trennwirksamen Einbauten ist ein zusätzlicher Flüssigkeitsverteiler oberhalb derselben nicht zwingend erforderlich; es ist auch möglich, dass die obersten Böden die Funktion desselben übernehmen, diese stehen dann jedoch nicht mehr für die eigentliche Trennaufgabe zur Verfügung.

Der Flüssigkeitsverteiler verteilt die Flüssigkeit gleichmäßig über den Kolonnenquerschnitt, und weist Einrichtungen auf, dergestalt, dass der Dampf getrennt von der Flüssigkeit nach oben durch die Kolonne geleitet wird. Als Flüssigkeitsverteiler können zum Beispiel Kastenrinnenverteiler, Lochbodenverteiler, Tüllenverteiler oder Rohrverteiler eingesetzt werden.

Der Rücklauf wird bevorzugt auf trennwirksame Einbauten, insbesondere Böden, aufgegeben, um das Lösungsmittel aus dem aufsteigenden Dampf auszuwaschen.

Die Vermischung der beiden Flüssigkeitsströme ist umso schwieriger, je größer die Unterschiede in der Dichte und der Viskosität sind. Dies trifft insbesondere für Dichteunterschiede von größer als etwa 3 bis 5 % und Viskositätsunterschiede von größer als etwa 50 % zu.

Problematisch ist beispielsweise die Vermischung von sogenanntem Raffinat 1, das heißt einer Mischung, die im Wesentlichen Butane und Butene enthält, mit N-Methylpyrrolidon und 8,3 Gew.-% Wasser als selektivem Lösungsmittel, da die beiden Flüssigkeiten sich sowohl in der Dichte (NMP/Wasser = 1.014 kg/m³ gegenüber Raffinat 1 = 572 kg/m³) sowie der Viskosität (NMP/Wasser = 1,179 mPa·s gegenüber Raffinat 1 = 0,14 mPa·s) deutlich unterscheiden.

Durch die besondere Ausgestaltung des ersten, im Wesentlichen horizontalen Einleitrohres, mit einer Querschnittsverengung bis zu einer engsten Stelle, und einer anschließenden Aufweitung, wird der bekannte Venturi-Effekt genutzt, das heißt die Einschnürung zeugt lokal erhöhte Geschwindigkeiten, wodurch über das zweite Einleitrohr, das im Bereich der engsten Stelle der Querschnittsverengung, das heißt der Einschnürung, angeordnet ist, der zweite Flüssigkeitsstrom eingesaugt wird, ohne dass hierfür eine Förderpumpe oder ein statischer Druck erforderlich wäre. Durch Ausnutzung des Venturi-Effektes wird somit eine gute Durchmischung bei Flüssigkeitsströmen, ohne Einsatz bewegter Bauteile sowie ohne zusätzliche Bauhöhe, die zur Aufbringung eines statischen Druckes erforderlich wäre, ermöglicht.

Vorliegend soll das erste Einleitrohr im Wesentlichen horizontal in der Kolonne angeordnet sein, worunter verstanden wird, dass geringfügige fertigungs- und einbaubedingte Abweichungen von der Horizontalen, von bis zu 5 Grad oder auch von bis zu 10 Grad mit umfasst sind.

Vorteilhaft wird das Verhältnis des Querschnittes des ersten, im Wesentlichen horizontalen Einleitrohres vor der Querschnittsverengung zum Querschnitt des ersten, horizontalen Einleitrohres an der engsten Stelle der Querschnittsverengung dergestalt gewählt, dass der Druck innerhalb des ersten, im Wesentlichen horizontalen Einleitrohres an der engsten Stelle der Querschnittsverengung größer als der Druck außerhalb des ersten, im Wesentlichen horizontalen Einleitrohres in unmittelbarer Nähe der engsten Stelle der Querschnittsverengung der engsten Stelle der Querschnittsverengung ist.

Weiter bevorzugt wird der Durchmesser des ersten, im Wesentlichen horizontalen Einleitrohres vor der Querschnittsverengung dergestalt gewählt, dass die Strömungsgeschwindigkeit im ersten, im Wesentlichen horizontalen Einleitrohr vor der Querschnittsverengung im Bereich von 0,1 bis 5,0 m/s, bevorzugt im Bereich von 0,3 bis 1,5 m/s liegt.

Die Geometrie des ersten, im Wesentlichen horizontalen Einleitrohres wird bevorzugt der Gestalt gewählt, dass sich die Querschnittsverengung über eine Länge von bis zu 4 mal dem Durchmesser des ersten, im Wesentlichen horizontalen Einleitrohres, bevorzugt von bis zu 3 mal und die Aufweitung nach der engsten Stelle der Querschnittsverengung, über eine Länge zwischen 0,1 mal den Durchmesser des ersten, im Wesentlichen horizontalen Einleitrohres und 15 mal den Durchmesser des ersten, im Wesentlichen horizontalen Einleitrohres, erstreckt.

Durch diese besondere Ausgestaltung des ersten, im Wesentlichen horizontalen Einleitrohres soll eine minimierte Länge desselben erreicht werden, so dass der Einbauaufwand hierfür möglichst niedrig ist.

In einer vorteilhaften Ausgestaltung taucht das zweite Einleitrohr, das im Bereich der engsten Stelle der Querschnittsverengung in das erste, im Wesentlichen horizontale Einleitrohr einmündet, in dasselbe, bevorzugt bis auf eine Eintauchtiefe von 0,1 mal bis 0,8 mal dem Durchmesser des zweiten Einleitrohres, bevorzugt bis auf eine Eintauchtiefe von 0,15 bis 0,75 mal dem Durchmesser des zweiten Einleitrohres, ein.

Durch diese bevorzugte Ausgestaltung des zweiten Einleitrohres wird eine zusätzliche Verbesserung der Vermischung über den Querschnitt des ersten, im Wesentlichen horizontalen Einleitrohres, erreicht. Weiter bevorzugt endet das zweite, in das erste, im Wesentlichen horizontale Rohr eingetauchte Einleitrohr abgeschrägt in einem Anstellwinkel gegenüber der Längsachse desselben in einem Bereich von 4° bis 65°.

Durch diese weiter bevorzugte Ausgestaltung wird eine weitere Verbesserung der Vermischung über den Querschnitt des ersten, im Wesentlichen horizontalen Einleitrohres durch verstärkte Ablösewirbel am Ende des zweiten Einleitrohres und insgesamt höhere Einsaugwirkung erreicht.

In einer weiter verbesserten Ausgestaltung ist im ersten, im Wesentlichen horizontalen Einleitrohr nach der engsten Stelle der Querschnittsverengung, im Wesentlichen quer zur Längsachse desselben, ein im Wesentlichen planes statisches Mischelement angeordnet, das den Querschnitt desselben teilweise blockiert.

Das vorliegend vorgesehene statische Mischelement soll im Wesentlichen plan sein, das heißt seine Abmessung in Längsrichtung des ersten, im Wesentlichen horizontalen Einleitrohres, soll gegenüber den beiden anderen Dimensionen desselben vernachlässigbar sein.

Das im Wesentlichen plane statische Mischelement soll den Querschnitt des ersten, im Wesentlichen horizontalen Einleitrohres teilweise, bevorzugt im Bereich von 5 % bis 50 % desselben blockieren.

Das im Wesentlichen plane statische Mischelement ist bevorzugt um mindestens das Doppelte des Durchmessers des ersten, im Wesentlichen horizontale Einleitrohres an der Stelle des engsten Querschnittes desselben von der Stelle des engsten Querschnittes beabstandet.

Vorteilhaft ist das statische Mischelement exzentrisch im Querschnitt des ersten, im Wesentlichen horizontalen Einleitrohres angeordnet, wobei es an der Innenwand desselben anliegt oder wandnah zu derselben ausgebildet ist, im oberen Bereich des ersten, im Wesentlichen horizontalen Einleitrohres jedoch von der Innenwand desselben beabstandet ist.

Bevorzugt ist das exzentrisch, im ersten, im Wesentlichen horizontalen Einleitrohr angeordnete statische Mischelement in Form eines Kreisringes ausgebildet.

In einer weiteren bevorzugten Ausführungsform ist das exzentrisch, im ersten, im Wesentlichen horizontalen Einleitrohr angeordnete statische Mischelement in Form eines nach oben offenen Kreisringes ausgebildet, der bevorzugt mittels Stegen im oberen Bereich der Innenwand an derselben befestigt ist.

Gegenstand der Erfindung ist auch ein Verfahren zur Auftrennung eines Gemisches von Kohlenwasserstoffen und/oder Kohlenwasserstoffderivaten durch Extraktivdestillation mit einem selektiven Lösungsmittel in einer Kolonne, mit Zuführung des selektiven Lösungsmittels im oberen Kolonnenbereich und Zuführung des aufzutrennenden Gemisches von Kohlenwasserstoffen und/oder Kohlenwasserstoffderivaten unterhalb der Zuführung des selektiven Lösungsmittels, wobei sich in der Kolonne das selektive Lösungsmittel mit den Komponenten aus dem aufzutrennenden Gemisch belädt, zu dem es eine höhere Affinität hat, und als beladenes selektives Lösungsmittel aus dem unteren Kolonnenbereich abgezogen wird, wogegen die Komponenten aus dem aufzutrennenden Gemisch von Kohlenwasserstoffen und/oder Kohlenwasserstoffderivaten, zu denen das selektive Lösungsmittel eine niedrigere Affinität hat, in der Dampfphase verbleiben und als Kopfstrom abgezogen werden, der vollständig oder teilweise unter Erhalt eines Kondensats kondensiert wird, das teilweise als Produktstrom abgezogen und im Übrigen als Rücklauf erneut auf die Kolonne aufgegeben wird, das dadurch gekennzeichnet ist, dass
- das selektive Lösungsmittel über ein erstes, im Wesentlichen horizontales Einleitrohr in den oberen Kolonnenbereich, oberhalb der trennwirksamen Einbauten, zugeführt wird, wobei das erste, im Wesentlichen horizontale Einleitrohr eine Querschnittsverengung bis zu einer engsten Stelle aufweist, und sich nach der Querschnittsverengung erneut aufweitet, und wobei
- über ein zweites Einleitrohr, das an der engsten Stelle der Querschnittsverengung in das erste, im Wesentlichen horizontale Einleitrohr einmündet, der Rücklauf zugeführt wird.

Bei dem erfindungsgemäßen Verfahren handelt es sich bevorzugt um die Extraktivdestillation von C₄-Schnitten zur Gewinnung von Butanen und/oder Butenen und/oder 1,3-Butadien mit einem selektiven Lösungsmittel ausgewählt aus N-Methylpyrrolidon oder Mischungen hiervon mit Wasser, Dimethylformamid und Acetonitril oder die Extraktivdestillation von aromatenhaltigen Gemischen zur Gewinnung von Benzol und/oder Toluol und/oder Xylol.

Die Erfindung wird im Folgenden anhand einer Zeichnung sowie von Ausführungsbeispielen näher erläutert.

In der Zeichnung zeigen im Einzelnen:
- Figur 1: die schematische Darstellung einer bevorzugten Ausführungsform einer erfindungsgemäßen Kolonne,
- Figur 2: die schematische Darstellung einer bevorzugten Ausführungsform für ein erstes, im Wesentlichen horizontal in der Kolonne angeordnetes Einleitrohr im Bereich der engsten Stelle der Querschnittsverengung im ersten, im Wesentlichen horizontalen Einleitrohr sowie mit einem statischen Mischelement stromabwärts der engsten Stelle der Querschnittsverengung im ersten, im Wesentlichen horizontalen Einleitrohr und
- Figuren 3A und 3B: Beispiele für bevorzugte Ausführungsformen statischer Mischelemente

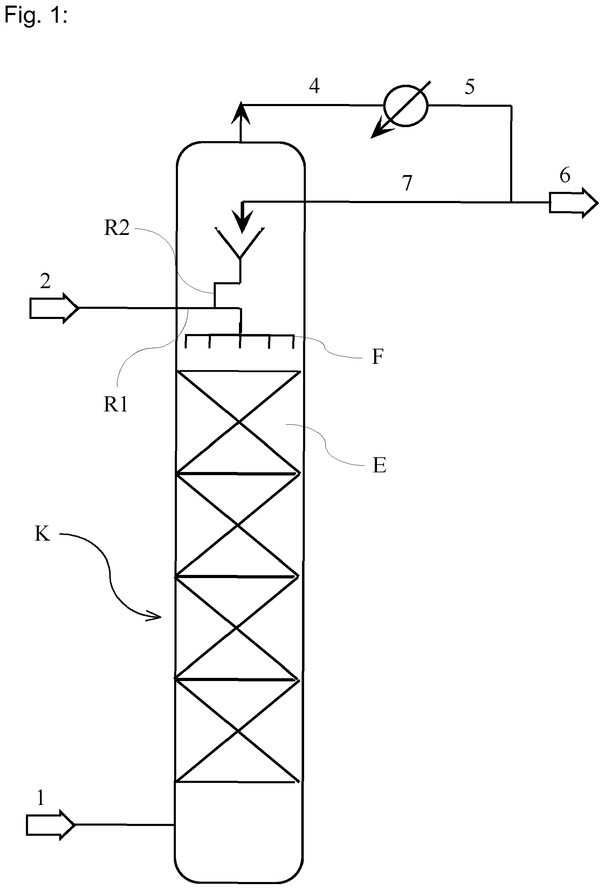

Figur 1 zeigt eine bevorzugte Ausführungsform für eine erfindungsgemäße Kolonne K für die Durchführung einer Extraktivdestillation eines Gemisches von Kohlenwasserstoffen und/oder Kohlenwasserstoffderivaten 1 durch Gegenstromführung mit einem selektiven Lösungsmittel 2 an trennwirksamen Einbauten E, mit einem Flüssigkeitsverteiler F oberhalb derselben, wobei sich in der Kolonne K das selektiven Lösungsmittel 2 mit den Komponenten aus dem aufzutrennenden Gemisch 1 belädt, zu denen es eine höhere Affinität hat, und als beladenes selektives Lösungsmittel 3 aus dem unteren Kolonnenbereich abgezogen wird, wogegen die Komponenten aus dem aufzutrennenden Gemisch, zu denen das selektive Lösungsmittel 2 eine niedrigere Affinität hat, in der Dampfphase verbleiben und als Kopfstrom 4 abgezogen werden, der unter Erhalt eines Kondensats 5 kondensiert wird, das teilweise als Produktstrom 6 abgezogen und im Übrigen als Rücklauf 7 erneut auf die Kolonne K aufgegeben wird.

Das selektive Lösungsmittel 2 wird über ein im Wesentlichen horizontales Einleitrohr R1 oberhalb der Einbauten E und des Flüssigkeitsverteilers F in die Kolonne eingeleitet. Das im Wesentlichen horizontales Einleitrohr R1 weist eine Geometrie auf, die einen Venturi-Effekt bewirkt, das heißt das Einleitrohr R1 hat eine Querschnittsverengung bis zu einer engsten Stelle, worauf es sich erneut aufweitet, und wobei im Bereich der engsten Stelle der Querschnittsverengung aus einem zweiten Einleitrohr R2 der Rücklauf 7 eingesaugt wird, ohne dass hierfür eine Förderpumpe oder ein statischer Druck erforderlich wäre.

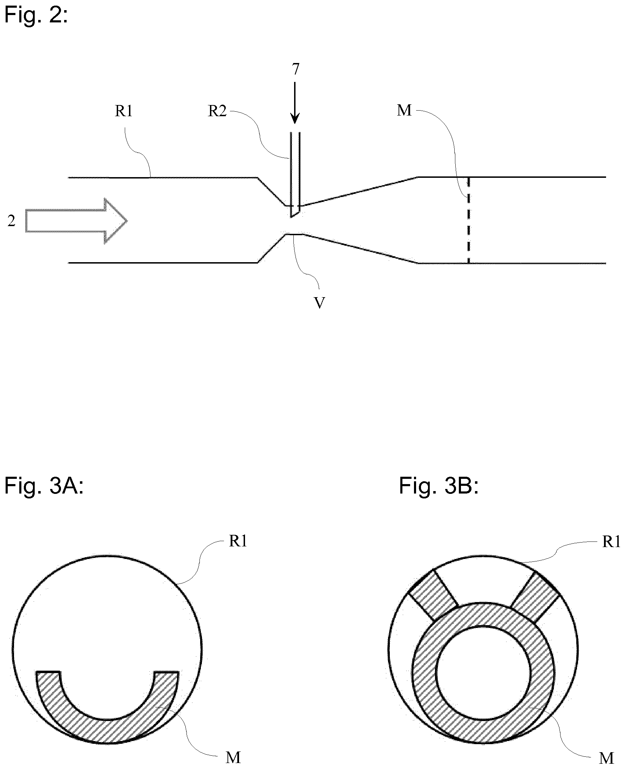

Die schematische Darstellung in Figur 2 verdeutlicht das für die Erfindung wesentliche Einmischelement des Rücklaufs 7 in das selektive Lösungsmittel 2: Das selektive Lösungsmittel 2 wird über ein erstes Einleitrohr R1, das eine Querschnittsverengung bis zu einem engsten Querschnitt V aufweist und sich anschließend erneut aufweitet, zugeführt. Im Bereich der engsten Stelle V der Querschnittsverengung ist das zweite Einleitrohr R2 angeordnet, durch das der Rücklauf 7 geleitet und in den Strom 2 des selektiven Lösungsmittels eingemischt wird. In der dargestellten bevorzugten Ausführungsvariante wird eine zusätzliche Verbesserung der Mischgüte erreicht, indem stromabwärts der engsten Stelle V der Querschnittsverengung und quer zur Hauptströmungsrichtung durch das erste Einleitrohr R1 ein statischer Mischer M angeordnet ist.

Bevorzugte geometrische Ausgestaltungen für statische Mischer M sind in den Figuren 3A und 3B dargestellt: in Form eines nach oben offenen Kreisringes, der an der unteren Innenwand des Rohres R1 anliegt, in Figur 3A bzw. in Form eines exzentrisch angeordneten Kreisringes, der ebenfalls an der unteren Innenwand des ersten Einleitrohres R1 anliegt, von der oberen Innenwand desselben jedoch beabstandet und daran mit Stegen befestigt ist, in Figur 3B.

### Ausführungsbeispiele:

In einer Extraktivdestillationskolonne K mit einem Innendurchmesser von 5,33 m wird oberhalb von trennwirksamen Einbauten E ein Strom von selektivem Lösungsmittel 2 enthaltend ein N-Methyl-Pyrrolidon/Wasser-Gemisch mit einer Dichte 1013,7 kg/m³ und einer Viskosität von 1,179 mPa·s, mit einem Massenstrom von 417 t/h eingeleitet. Der Rücklauf 7 mit einem Massenstrom von 22 t/h enthält ein Gemisch aus Butanen und Butenen mit einer Dichte von 572,1 kg/m³ und einer Viskosität von 0,14 mPa·s.

### Vergleichsbeispiel:

Zum Vergleich ist in der Extraktivdestillationskolonne K oberhalb der trennwirksamen Einbauen E ein handelsüblicher Lochboden-Flüssigkeitsverteiler angeordnet. In der Ebene der Öffnungen des Flüssigkeitsaustritts aus dem Flüssigkeitsverteiler beträgt die Mischgüte Xₘₐₓ/Xₐᵥ, die wie nachfolgend angegeben definiert wird, 6,8.

Bei der Angabe der Mischgüte bezeichnet Xₘₐₓ vorliegend den höchsten Wert im Messgebiet für den Massenanteil des Stromes 7,
Xₐᵥ bezeichnet entsprechend den mittleren Wert im Messgebiet für den Massenanteil des Stromes 7, das heißt den Wert, der bei perfekter Mischung im gesamten Messgebiet zu finden ist.

Die Mischgüte wird durch das Verhältnis Xₘₐₓ/Xₐᵥ definiert. Sie ist somit 1 bei idealer Vermischung.

Analog wird das Verhältnis Xₘᵢₙ/Xₐᵥ definiert, das heißt als Verhältnis aus dem niedrigsten Wert im Messgebiet, bezogen auf den mittleren Wert im Messgebiet, jeweils für den Massenanteil des Stromes 7.

### Erfindungsgemäße Beispiele:

### Beispiel 1:

Einmischvorrichtung oberhalb der trennwirksamen Einbauten E ist ein erstes, im Wesentlichen horizontal angeordnetes Einleitrohr R1 vorgesehen, das eine Querschnittsverengung bis zu einer engsten Stelle V aufweist und sich danach erneut aufweitet. Im Bereich der engsten Stelle V der Querschnittsverengung V mündet ein zweites Einleitrohr R2 in dieses ein, taucht teilweise in das erste, im Wesentlichen horizontale Einleitrohr R1 ein und weist ein abgeschrägtes Ende auf.

Die konkreten Abmessungen sind die Folgenden:
- Durchmesser des ersten, im Wesentlichen horizontalen Einleitrohres R1 = 304,8 mm,
- Querschnittsverengung des ersten, im Wesentlichen horizontalen Einleitrohres R1 über eine Länge von 150 mm in Längsrichtung derselben,
- Bereich der engsten Stelle V der Querschnittsverengung: über 75 mm in Längsrichtung des ersten, im Wesentlichen horizontalen Einleitrohres R1,
- Durchmesser an der engsten Stelle V des ersten, im Wesentlichen horizontalen Einleitrohres R1 = 130 mm,
- woran sich eine Aufweitung bis auf den ursprünglichen Durchmesser, von 304,8 mm über eine Länge von 480 mm des ersten, im Wesentlichen horizontalen Einleitrohres R1, erstreckt.

Im Bereich der engsten Stelle V der Querschnittsverengung taucht ein zweites Einleitrohr R2, mit einem Innendurchmesser von 50,8 mm in das erste, im Wesentlichen horizontale Einleitrohres R1, und zwar bis auf eine Eintauchtiefe von 49,2 mm am stromaufwärts gelegenen Ende bzw. bis auf eine Eintauchtiefe von 29,2 mm am stromabwärts gelegenen Ende desselben ein, das heißt, das zweite Einleitrohr R2 ist abgeschrägt.

In einem Messgebiet, das als Querschnitt des ersten, im Wesentlichen horizontalen Einleitrohres R1, in einer Entfernung von 2 m stromabwärts des stromabwärts gerichteten Endes der engsten Stelle V der Querschnittsverengung definiert wird, werden die folgenden Werte für die Mischgüte ermittelt:
Xₘₐₓ/Xₐᵥ gleich 1,09 und
Xₘᵢₙ/Xₐᵥ gleich 0,85.

### Beispiel 2:

Die Anordnung in Beispiel 2 entspricht der Anordnung in Beispiel 1, wobei jedoch zusätzlich stromabwärts der Einleitrohre R1 und R2 ein statisches Mischelement M angeordnet ist, entsprechend der schematischen Darstellung in Figur 3B in Form eines exzentrisch angeordneten Ringes aus einem Stahlblech mit einer Dicke von 4 mm, einem Außendurchmesser von 260 mm und einem Innendurchmesser von 200 mm, das an der unteren Innenwand des ersten, im Wesentlichen horizontalen Einleitrohres R1 anliegt und an der oberen Innenwand desselben mit zwei Stegen befestigt ist.

Die Mischgüte ermittelt sich in diesem Fall zu 1,02 für Xₘₐₓ/Xₐᵥ bzw. 0,99 für Xₘᵢₙ/Xₐᵥ.

### Bezugszeichenliste

### Stoffströme:

- 1: Gemisch von Kohlenwasserstoffen und/oder Kohlenwasserstoffderivaten
- 2: selektives Lösungsmittel
- 3: beladenes, selektives Lösungsmittel
- 4: Kopfstrom
- 5: Kondensat
- 6: Produktstrom
- 7: Rücklauf

### Apparate und -teile:

- K: Kolonne
- E: trennwirksame Einbauten
- F: Flüssigkeitsverteiler
- R1: erstes, im Wesentlichen horizontales Einleitrohr
- R2: zweites Einleitrohr
- V: engste Stelle der Querschnittsverengung

## Patentansprüche

1. Kolonne (K) mit trennwirksamen Einbauten (E) zur Auftrennung eines Gemisches von Kohlenwasserstoffen und/oder Kohlenwasserstoffderivaten (1) durch Extraktivdestillation mit einem selektiven Lösungsmittel (2),
mit Zuführung des selektiven Lösungsmittels (2) im oberen Kolonnenbereich und Zuführung des aufzutrennenden Gemisches von Kohlenwasserstoffen und/oder Kohlenwasserstoffderivaten (1) unterhalb der Zuführung des selektiven Lösungsmittels (2), wobei sich in der Kolonne (K) das selektive Lösungsmittel (2) mit den Komponenten aus dem aufzutrennenden Gemisch (1) belädt, zu denen es eine höhere Affinität hat, und als beladenes selektives Lösungsmittel (3) aus dem unteren Kolonnenbereich abgezogen wird,
wogegen die Komponenten aus dem aufzutrennenden Gemisch, zu denen das selektive Lösungsmittel (2) eine niedrigere Affinität hat, in der Dampfphase verbleiben und als Kopfstrom (4) abgezogen werden,
der vollständig oder teilweise unter Erhalt eines Kondensats (5) kondensiert wird, das
teilweise als Produktstrom (6) abgezogen und im Übrigen als Rücklauf (7) erneut auf die Kolonne (K) aufgegeben wird,
**dadurch gekennzeichnet, dass**
- im Kolonnenbereich oberhalb der trennwirksamen Einbauten (E), ein erstes, im Wesentlichen horizontales Einleitrohr (R1) für die Zuführung des selektiven Lösungsmittels vorgesehen ist,
- wobei das erste, im Wesentlichen horizontale Einleitrohr (R1) eine Querschnittsverengung bis zu einer engsten Stelle (V) aufweist, und sich nach der Querschnittsverengung erneut aufweitet, und wobei
- ein zweites Einleitrohr (R2), das im Bereich der engsten Stelle der Querschnittsverengung (V) in das erste, im Wesentlichen horizontale Einleitrohr (R1) einmündet, für die Zuführung des Rücklaufs (7) vorgesehen ist.

2. Kolonne (K) nach Anspruch 1, **dadurch gekennzeichnet, dass** oberhalb der trennwirksamen Einbauten (E) in der Kolonne (K) ein Flüssigkeitsverteiler (F) angeordnet ist.

3. Kolonne (K) nach Anspruch 1 oder 2, **dadurch gekennzeichnet, dass** sie einen Durchmesser von > 0,5 m, insbesondere einen Durchmesser von > 1,0 m aufweist.

4. Kolonne (K) nach einem der Ansprüche 1 bis 3, **dadurch gekennzeichnet, dass** das Verhältnis des Querschnittes des ersten, im Wesentlichen horizontalen Einleitrohres (R1) vor der Querschnittsverengung zum Querschnitt des ersten, horizontalen Einleitrohres (R1) an der engsten Stelle (V) der Querschnittsverengung dergestalt gewählt wird, dass der Druck innerhalb des ersten, im Wesentlichen horizontalen Einleitrohres (R1) an der engsten Stelle (V) der Querschnittsverengung kleiner als der Druck außerhalb des ersten, im Wesentlichen horizontalen Einleitrohres (R1) in unmittelbarer Nähe der engsten Stelle (V) der Querschnittsverengung der engsten Stelle (V) der Querschnittsverengung ist.

5. Kolonne (K) nach einem der Ansprüche 1 bis 4, **dadurch gekennzeichnet, dass** der Durchmesser des ersten, im Wesentlichen horizontalen Einleitrohres (R1) vor der Querschnittsverengung dergestalt gewählt wird, dass sich die Strömungsgeschwindigkeit im ersten, im Wesentlichen horizontalen Einleitrohr (R1) vor der Querschnittsverengung im Bereich von 0,1 bis 5,0 m/s, bevorzugt im Bereich von 0,3 bis 1,5 m/s, liegt.

6. Kolonne (K) nach einem der Ansprüche 1 bis 5, **dadurch gekennzeichnet, dass** das zweite Einleitrohr (R2), das im Bereich der engsten Stelle (V) der Querschnittsverengung in das erste, im Wesentlichen horizontale Einleitrohr (R1) einmündet, in dasselbe, bevorzugt bis auf eine Eintauchtiefe von 0,1 mal bis 0,8 mal dem Durchmesser des zweiten Einleitrohres (R2), bevorzugt bis auf eine Eintauchtiefe von 0,15 bis 0,75 mal dem Durchmesser des zweiten Einleitrohres (R2), eintaucht.

7. Kolonne (K) nach Anspruch 6, **dadurch gekennzeichnet, dass** das zweite, in das erste, im Wesentlichen horizontale Rohr eingetauchte Einleitrohr abgeschrägt in einem Anstellwinkel gegenüber der Längsachse desselben in einem Bereich von 4° bis 65° endet.

8. Kolonne (K) nach einem der Ansprüche 1 bis 7, **dadurch gekennzeichnet, dass** im ersten, im Wesentlichen horizontalen Einleitrohr, nach der engsten Stelle der Querschnittsverengung, im Wesentlichen quer zur Längsachse desselben, ein statisches Mischelement (M) angeordnet ist, das den Querschnitt desselben teilweise blockiert.

9. Kolonne (K) nach Anspruch 8, **dadurch gekennzeichnet, dass** das statische Mischelement (M) um mindestens das Doppelte des Durchmessers des ersten, im Wesentlichen horizontalen Einleitrohres (R1) an der Stelle des engsten Querschnittes (V) von der Stelle des engsten Querschnittes (V) im ersten, im Wesentlichen horizontalen Einleitrohr(R1) beabstandet ist.

10. Kolonne (K) nach Anspruch 8 oder 9, **dadurch gekennzeichnet, dass** das statische Mischelement (M) exzentrisch im Querschnitt des ersten, im Wesentlichen horizontalen Einleitrohres (R1) angeordnet ist, wobei es an der Innenwand desselben anliegt oder wandnah zu derselben ausgebildet, im oberen Bereich des ersten, im Wesentlichen horizontalen Einleitrohres (R1) jedoch von der Innenwand desselben beabstandet ist.

11. Kolonne (K) nach Anspruch 10, **dadurch gekennzeichnet, dass** das exzentrisch, im ersten, im Wesentlichen horizontalen Einleitrohr (R1) angeordnete statische Mischelement (M) in Form eines Kreisringes ausgebildet ist.

12. Kolonne (K) nach Anspruch 11, **dadurch gekennzeichnet, dass** das exzentrisch, im ersten, im Wesentlichen horizontalen Einleitrohr (R1) angeordnete statische Mischelement (M) in Form eines nach oben offenen Kreisringes ausgebildet ist, der bevorzugt mittels Stegen im oberen Bereich der Innenwand an derselben befestigt ist.

13. Verfahren zur Auftrennung eines Gemisches von Kohlenwasserstoffen und/oder Kohlenwasserstoffderivaten (1) durch Extraktivdestillation mit einem selektiven Lösungsmittel (2) in einer Kolonne (K) nach einem der Ansprüche 1 bis 12,
mit Zuführung des selektiven Lösungsmittels (2) im oberen Kolonnenbereich und Zuführung des aufzutrennenden Gemisches von Kohlenwasserstoffen und/oder Kohlenwasserstoffderivaten (1) unterhalb der Zuführung des selektiven Lösungsmittels (2), wobei sich in der Kolonne (K) das selektive Lösungsmittel (2) mit den Komponenten aus dem aufzutrennenden Gemisch belädt, zu dem es eine höhere Affinität hat, und als beladenes selektives Lösungsmittel (3) aus dem unteren Kolonnenbereich abgezogen wird,
wogegen die Komponenten aus dem aufzutrennenden Gemisch von Kohlenwasserstoffen und/oder Kohlenwasserstoffderivaten (1), zu denen das selektive Lösungsmittel (2) eine niedrigere Affinität hat, in der Dampfphase verbleiben und als Kopfstrom (4) abgezogen werden,
der vollständig oder teilweise unter Erhalt eines Kondensats (5) kondensiert wird, das
teilweise als Produktstrom (6) abgezogen und im Übrigen als Rücklauf (7) erneut auf die Kolonne aufgegeben wird,
**dadurch gekennzeichnet, dass**
- das selektive Lösungsmittel (2) über ein erstes, im Wesentlichen horizontales Einleitrohr (R1) in den oberen Kolonnenbereich, oberhalb der trennwirksamen Einbauten (E), zugeführt wird, wobei das erste, im Wesentlichen horizontale Einleitrohr (R1) eine Querschnittsverengung bis zu einer engsten Stelle (V) aufweist, und sich nach der Querschnittsverengung erneut aufweitet, und wobei
- über ein zweites Einleitrohr (R2), das an der engsten Stelle (V) der Querschnittsverengung in das erste, im Wesentlichen horizontale Einleitrohr (R1) einmündet, der Rücklauf (7) zugeführt wird.

14. Verfahren nach Anspruch 13, **dadurch gekennzeichnet, dass** es sich um die Extraktivdestillation von C₄-Schnitten zur Gewinnung von Butanen und/oder Butenen und/oder 1,3-Butadien mit einem selektiven Lösungsmittel ausgewählt aus N-Methylpyrrolidon oder Mischungen hiervon mit Wasser, Dimethylformamid und Acetonitril oder die Extraktivdestillation von aromatenhaltigen Gemischen zur Gewinnung von Benzol und/oder Toluol und/oder Xylol, handelt.

## Claims

1. A column (K) comprising separatory internals (E) for separating a mixture of hydrocarbons and/or hydrocarbon derivatives (1) by extractive distillation with a selective solvent (2),
with supply of the selective solvent (2) in the upper region of the column and supply of the mixture of hydrocarbons and/or hydrocarbon derivatives to be separated (1) below the supply of the selective solvent (2), the selective solvent (2) becoming laden in the column (K) with the components from the mixture to be separated (1) for which it has greater affinity and being withdrawn from the lower region of the column as laden selective solvent (3),
while, by contrast, the components from the mixture to be separated for which the selective solvent (2) has a lower affinity remain in the vapor phase and are withdrawn as top stream (4),
which is completely or partially condensed to obtain a condensate (5),
some of which is withdrawn as product stream (6), the remainder being reintroduced to the column (K) as reflux (7),
wherein
- said column comprises in the region of the column above the separatory internals (E) a first, substantially horizontal feed pipe (R1) for supplying the selective solvent,
- wherein the first, substantially horizontal feed pipe (R1) exhibits a cross-sectional narrowing to a narrowest point (V), said pipe widening again downstream of the cross-sectional narrowing, and wherein
- said column comprises a second feed pipe (R2) for supplying the reflux (7), said pipe joining the first, substantially horizontal feed pipe (R1) in the region of the narrowest point (V) of the cross-sectional narrowing.

2. The column (K) according to claim 1, wherein there is a liquid distributor (F) disposed above the separatory internals (E) in the column (K).

3. The column (K) according to either of claims 1 and 2, wherein said column has a diameter of > 0.5 m, in particular a diameter of > 1.0 m.

4. The column (K) according to any of claims 1 to 3, wherein the ratio of the cross section of the first, substantially horizontal feed pipe (R1) upstream of the cross-sectional narrowing to the cross section of the first, horizontal feed pipe (R1) at the narrowest point (V) of the cross-sectional narrowing is chosen such that the pressure inside the first, substantially horizontal feed pipe (R1) at the narrowest point (V) of the cross-sectional narrowing is lower than the pressure outside the first, substantially horizontal feed pipe (R1) immediately proximal to the narrowest point (V) of the cross-sectional narrowing.

5. The column (K) according to any of claims 1 to 4, wherein the diameter of the first, substantially horizontal feed pipe (R1) upstream of the cross-sectional narrowing is chosen such that the flow velocity in the first, substantially horizontal feed pipe (R1) upstream of the cross-sectional narrowing is in the range of from 0.1 to 5.0 m/s, preferably in the range of from 0.3 to 1.5 m/s.

6. The column (K) according to any of claims 1 to 5, wherein the second feed pipe (R2) that joins the first, substantially horizontal feed pipe (R1) in the region of the narrowest point (V) of the cross-sectional narrowing preferably protrudes into said pipe by a protrusion depth of from 0.1 to 0.8 times the diameter of the second feed pipe (R2), preferably by a protrusion depth of from 0.15 to 0.75 times the diameter of the second feed pipe (R2).

7. The column (K) according to claim 6, wherein the second feed pipe protruding into the first, substantially horizontal pipe terminates slantedly at an angle to the longitudinal axis of said second feed pipe in the range of from 4° to 65°.

8. The column (K) according to any of claims 1 to 7, wherein the first, substantially horizontal feed pipe has disposed in it, downstream of the narrowest point of the cross-sectional narrowing and substantially transversely to the longitudinal axis of said pipe, a static mixing element (M) that partially blocks the cross section of said pipe.

9. The column (K) according to claim 8, wherein the static mixing element (M) is spaced apart from the point of the narrowest cross section (V) in the first, substantially horizontal feed pipe (R1) by at least double the diameter of the first, substantially horizontal feed pipe (R1) at the point of the narrowest cross section (V).

10. The column (K) according to either of claims 8 and 9, wherein the static mixing element (M) is eccentrically disposed in the cross section of the first, substantially horizontal feed pipe (R1) and is in contact with the interior wall of said pipe or is close to the wall thereof but in the upper region of the first, substantially horizontal feed pipe (R1) is spaced apart from the interior wall of said pipe.

11. The column (K) according to claim 10, wherein the static mixing element (M) eccentrically disposed in the first, substantially horizontal feed pipe (R1) is in the shape of an annulus.

12. The column (K) according to claim 11, wherein the static mixing element (M) eccentrically disposed in the first, substantially horizontal feed pipe (R1) is in the shape of an annulus which is open at the top, said annulus preferably being secured to the interior wall by means of supports in the upper region of said interior wall.

13. A process for separating a mixture of hydrocarbons and/or hydrocarbon derivatives (1) by extractive distillation with a selective solvent (2) in a column (K) according to any of claims 1 to 12,
with supply of the selective solvent (2) in the upper region of the column and supply of the mixture of hydrocarbons and/or hydrocarbon derivatives to be separated (1) below the supply of the selective solvent (2), the selective solvent (2) becoming laden in the column (K) with the components from the mixture to be separated for which it has greater affinity and being withdrawn from the lower region of the column as laden selective solvent (3),
while, by contrast, the components from the mixture of hydrocarbons and/or hydrocarbon derivatives (1) to be separated for which the selective solvent (2) has a lower affinity remain in the vapor phase and are withdrawn as top stream (4),
which is completely or partially condensed to obtain a condensate (5),
some of which is withdrawn as product stream (6), the remainder being reintroduced to the column as reflux (7),
wherein
- the selective solvent (2) is supplied into the upper region of the column above the separatory internals (E) via a first, substantially horizontal feed pipe (R1), wherein the first, substantially horizontal feed pipe (R1) exhibits a cross-sectional narrowing to a narrowest point (V), said pipe widening again downstream of the cross-sectional narrowing, and wherein
- the reflux (7) is supplied via a second feed pipe (R2) which joins the first, substantially horizontal feed pipe (R1) at the narrowest point (V) of the cross-sectional narrowing.

14. The process according to claim 13, wherein said process is an extractive distillation of C4 cuts to obtain butanes and/or butenes and/or 1,3-butadiene with a selective solvent selected from N-methylpyrrolidone or mixtures thereof with water, dimethylformamide and acetonitrile or an extractive distillation of aromatics-containing mixtures to obtain benzene and/or toluene and/or xylene.

## Revendications

1. Colonne (K) comprenant des composants internes de séparation (E) pour la séparation d'un mélange d'hydrocarbures et/ou de dérivés d'hydrocarbures (1) par distillation extractive avec un solvant sélectif (2), avec introduction du solvant sélectif (2) dans la zone supérieure de la colonne et introduction du mélange d'hydrocarbures et/ou de dérivés d'hydrocarbures à séparer (1) en dessous de l'introduction du solvant sélectif (2), le solvant sélectif (2) se chargeant dans la colonne (K) avec les composants du mélange à séparer (1) avec lesquels il a une affinité plus élevée, et étant soutiré de la zone inférieure de la colonne en tant que solvant sélectif chargé (3),
tandis que les composants du mélange à séparer avec lesquels le solvant sélectif (2) a une affinité plus faible restent dans la phase vapeur et sont soutirés en tant que courant de tête (4),
qui est condensé en totalité ou en partie pour obtenir un condensat (5),
qui est soutiré en partie en tant que courant de produits (6) et pour le reste réintroduit en tant que reflux (7) dans la colonne (K),
**caractérisé en ce que**
- dans la zone de colonne au-dessus des composants internes de séparation (E), un premier tube d'introduction essentiellement horizontal (R1) est prévu pour l'introduction du solvant sélectif,
- le premier tube d'introduction essentiellement horizontal (R1) présentant un rétrécissement de la section transversale jusqu'à un emplacement le plus étroit (V), et s'élargissant de nouveau après le rétrécissement de section transversale, et
- un deuxième tube d'introduction (R2), qui débouche dans la zone de l'emplacement le plus étroit du rétrécissement de section transversale (V) dans le premier tube d'introduction essentiellement horizontal (R1), étant prévu pour l'introduction du reflux (7).

2. Colonne (K) selon la revendication 1, **caractérisée en ce qu'**un distributeur de liquide (F) est agencé au-dessus des composants internes de séparation (E) dans la colonne (K).

3. Colonne (K) selon la revendication 1 ou 2, **caractérisée en ce qu'**elle présente un diamètre de > 0,5 m, notamment un diamètre de > 1,0 m.

4. Colonne (K) selon l'une quelconque des revendications 1 à 3, **caractérisée en ce que** le rapport entre la section transversale du premier tube d'introduction essentiellement horizontal (R1) avant le rétrécissement de section transversale et la section transversale du premier tube d'introduction essentiellement horizontal (R1) au niveau de l'emplacement le plus étroit (V) du rétrécissement de section transversale est choisi de telle sorte que la pression dans le premier tube d'introduction essentiellement horizontal (R1) au niveau de l'emplacement le plus étroit (V) du rétrécissement de section transversale soit plus faible que la pression à l'extérieur du premier tube d'introduction essentiellement horizontal (R1) à proximité directe de l'emplacement le plus étroit (V) du rétrécissement de section transversale.

5. Colonne (K) selon l'une quelconque des revendications 1 à 4, **caractérisée en ce que** le diamètre du premier tube d'introduction essentiellement horizontal (R1) avant le rétrécissement de section transversale est choisi de telle sorte que la vitesse d'écoulement dans le premier tube d'introduction essentiellement horizontal (R1) avant le rétrécissement de section transversale se situe dans la plage allant de 0,1 à 5,0 m/s, de préférence dans la plage allant de 0,3 à 1,5 m/s.

6. Colonne (K) selon l'une quelconque des revendications 1 à 5, **caractérisée en ce que** le deuxième tube d'introduction (R2) qui débouche dans la zone de l'emplacement le plus étroit (V) du rétrécissement de section transversale dans le premier tube d'introduction essentiellement horizontal (R1) est immergé dans celui-ci de préférence jusqu'à une profondeur d'immersion de 0,1 fois à 0,8 fois le diamètre du deuxième tube d'introduction (R2), de préférence jusqu'à une profondeur d'immersion de 0,15 à 0,75 fois le diamètre du deuxième tube d'introduction (R2).

7. Colonne (K) selon la revendication 6, **caractérisée en ce que** le deuxième tube d'introduction immergé dans le premier tube essentiellement horizontal finit en biais à un angle d'incidence par rapport à l'axe longitudinal de celui-ci dans une plage allant de 4° à 65°.

8. Colonne (K) selon l'une quelconque des revendications 1 à 7, **caractérisée en ce que**, dans le premier tube d'introduction essentiellement horizontal, après l'emplacement le plus étroit du rétrécissement de section transversale, essentiellement perpendiculairement à l'axe longitudinal de celui-ci, un élément de mélange statique (M) est agencé, qui bloque en partie la section transversale de celui-ci.

9. Colonne (K) selon la revendication (8), **caractérisée en ce que** l'élément de mélange statique (M) est espacé au moins du double du diamètre du premier tube d'introduction essentiellement horizontal (R1) à l'emplacement de la section transversale la plus étroite (V) de l'emplacement de la section transversale la plus étroite (V) dans le premier tube d'introduction essentiellement horizontal (R1).

10. Colonne (K) selon la revendication 8 ou 9, **caractérisée en ce que** l'élément de mélange statique (M) est agencé excentriquement dans la section transversale du premier tube d'introduction essentiellement horizontal (R1), ledit élément de mélange étant adjacent à la paroi intérieur de celui-ci ou étant configuré à proximité de la paroi de celui-ci, dans la zone supérieure du premier tube d'introduction essentiellement horizontal (R1), mais toutefois espacé de la paroi intérieure de celui-ci.

11. Colonne (K) selon la revendication 10, **caractérisée en ce que** l'élément de mélange statique (M) agencé excentriquement dans le premier tube d'introduction essentiellement horizontal (R1) est configuré sous la forme d'un anneau circulaire.

12. Colonne (K) selon la revendication 11, **caractérisée en ce que** l'élément de mélange statique (M) agencé excentriquement dans le premier tube d'introduction essentiellement horizontal (R1) est configuré sous la forme d'un anneau circulaire ouvert vers le haut, qui est de préférence fixé au moyen de traverses dans la zone supérieure de la paroi intérieure de celui-ci.

13. Procédé de séparation d'un mélange d'hydrocarbures et/ou de dérivés d'hydrocarbures (1) par distillation extractive avec un solvant sélectif (2) dans une colonne (K) selon l'une quelconque des revendication 1 à 12, avec introduction du solvant sélectif (2) dans la zone supérieure de la colonne et introduction du mélange d'hydrocarbures et/ou de dérivés d'hydrocarbures à séparer (1) en dessous de l'introduction du solvant sélectif (2), le solvant sélectif (2) se chargeant dans la colonne (K) avec les composants du mélange à séparer avec lesquels il a une affinité plus élevée, et étant soutiré de la zone inférieure de la colonne en tant que solvant sélectif chargé (3),
tandis que les composants du mélange d'hydrocarbures et/ou de dérivés d'hydrocarbures à séparer (1) avec lesquels le solvant sélectif (2) a une affinité plus faible restent dans la phase vapeur et sont soutirés en tant que courant de tête (4),
qui est condensé en totalité ou en partie pour obtenir un condensat (5),
qui est soutiré en partie en tant que courant de produits (6) et pour le reste réintroduit en tant que reflux (7) dans la colonne (K),
**caractérisé en ce que**
- le solvant sélectif (2) est introduit par un premier tube d'introduction essentiellement horizontal (R1) dans la zone supérieure de la colonne, au-dessus des composants internes de séparation (E), le premier tube d'introduction essentiellement horizontal (R1) présentant un rétrécissement de la section transversale jusqu'à un emplacement le plus étroit (V), et s'élargissant de nouveau après le rétrécissement de section transversale, et
- le reflux (7) étant introduit par un deuxième tube d'introduction (R2), qui débouche au niveau de l'emplacement le plus étroit (V) du rétrécissement de section transversale dans le premier tube d'introduction essentiellement horizontal (R1).

14. Procédé selon la revendication 13, **caractérisé en ce qu'**il s'agit de la distillation extractive de coupes en C₄ pour l'obtention de butanes et/ou de butènes et/ou de 1,3-butadiène avec un solvant sélectif choisi parmi la N-méthylpyrrolidone ou des mélanges de celle-ci avec de l'eau, le diméthylformamide et l'acétonitrile, ou de la distillation extractive de mélanges contenant des composés aromatiques pour l'obtention de benzène et/ou de toluène et/ou de xylène.
